# EUROPEAN PATENT APPLICATION

(11) **EP 1 650 266 A1**
(43) Date of publication of application: **26.04.2006**
(21) Application number: 04746196.7
(22) Date of filing: 21.06.2004
(51) Int. Cl.: C08L 101/14, C08K 5/34, C08L 1/08, C07D 487/04, A61K 31/5517, A61K 47/38, A61K 47/48, A61P 37/08

(54) **PRODUCT OF COPRECIPITATION OF SPARINGLY SOLUBLE SUBSTANCE AND WATER-SOLUBLE POLYMER AND PROCESS FOR PRODUCING THE SAME**

(30) Priority: 20.06.2003 JP 2003175646
(71) Applicant: Meiji Seika Kaisha, Ltd., Tokyo 104-8002 (JP)
(72) Inventor: ISHIKURA, T., Chem. Manufacturing & Control Res., Kouhoku-ku, Yokohama-shi 222-8567 (JP); UDAGAWA, C., Chem. Manufacturing & Control Res., Kouhoku-ku, Yokohama-shi 222-8567 (JP); MISAKA, M., Chem. Manufacturing & Control Res., Kouhoku-ku, Yokohama-shi 222-8567 (JP); SUEMUNE, K., Chem. Manufacturing and Control Res., Kouhoku-ku, Yokohama-shi 222-8567 (JP); KITAHARA, S., Chem. Manufacturing & Control Res., Kouhoku-ku, Yokohama-shi 222-8567 (JP); ONO, K., Chem. Manufacturing & Control Res., Kouhoku-ku, Yokohama-shi 222-8567 (JP); KOYANAGI, A., Chem. Manufacturing & Control Res., Kouhoku-ku, Yokohama-shi 222-8567 (JP)
(74) Representative: Schrimpf, Robert
(86) International application number: PCT/JP2004/008727
(87) International publication number: WO 2004/113451

(57) **Abstract**

The present invention provides a coprecipitate of 2-(1-isopropoxycarbonyloxy-2-methylpropyl)-7,8-dimethoxy-4(5H),10-dio xo-2H-1,2,3-triazolo[4,5-c][1]benzazepine and a water-soluble polymer, excellent in solubility and absorbability.

## Description

### BACKGROUND OF THE INVENTION

Field of the Invention
The present invention relates to a coprecipitate of 2-(1-isopropoxycarbonyloxy-2-methylpropyl)-7,8-dimethoxy-4(5H),10-dio xo-2H-1,2,3-triazolo[4,5-c][1]benzazepine and a water-soluble polymer compound, excellent in solubility and absorbability, and to a process for producing the coprecipitate.

Background Art
2-(1-Isopropoxycarbonyloxy-2-methylpropyl)-7,8-dimethoxy-4(5H) ,10-dioxo-2H-1,2,3-triazolo[4,5-c][1]benzazepine (hereinafter referred to as "Compound A") has the following chemical formula. Since this compound, when orally administered, acts to stabilize mast cell membranes and to suppress allergic inflammation, it is expected to clinically use as oral antiallergic agents (WO 99/16770, Japanese Patent No. 3188482, and US Patent No. 6372735). However, since Compound A is sparingly soluble, when it is formulated into a preparation as it is, it is scarcely dissolved within the digestive tracts and is therefore poorly absorbed into organisms. In general, the absorbability of poorly absorbent compounds varies widely. It is, therefore, difficult to obtain the efficacy of such compounds with high reproducibility, and this fact can cause big clinical problems. For this reason, to design and produce oral preparations, it is desirable to improve the solubility and the absorbability of Compound A.

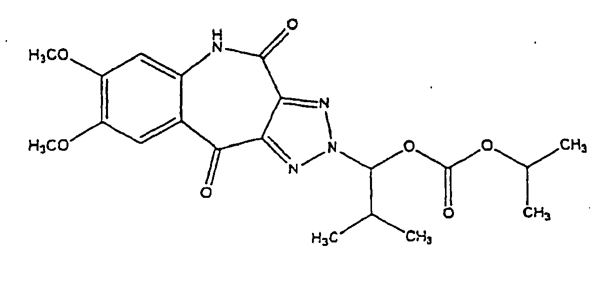

We have tried various methods to improve the solubility and the absorbability of Compound A. Since Compound A has, in its structure, no functional group that dissociates or is protonated in a pharmaceutically acceptable pH range, it has been difficult to dissolve Compound A by the use of an acidic or basic additive. Further, even when any of inclusion compounds such as cyclodextrins, a variety of surfactants, polymer compounds, and the like is added, it has been difficult to substantially solubilize Compound A. Furthermore, Compound A has not been dissolved in glycerin, propylene glycol, Macrogol 400, or the like to such a degree that the solution is pharmaceutically useful. In addition, we pulverized Compound A in accordance with the description in the reference (Japanese Patent Publication No. 185,013/1987) disclosing medicines that have been made readily absorbable by pulverization, and orally administered it to experimental animals such as dogs. We, however, confirmed that the absorbability of Compound A was not improved to such an extent that the use of Compound A for medicines could be expected. Moreover, we subjected Compound A to extruder treatment (WO94/08568; and Koichi Nakamichi, *et al., Yakuzai-gaku* (or Pharmaceutics), 56, 15-22 (1996)). The result was that Compound A was merely dispersed as crystals having low solubility or that Compound A was decomposed.

Amorphous state is known as a solid state excellent in solubility. An amorphous substance is in "a solid state which atoms (or molecules) have gathered without forming a crystal regularly structured in its special arrangement *(Iwanami Rikagaku Jiten,* 4^{th} ed., |wanami-Shoten, Japan, p. 1034 (1993)), and is defined as follows: "an amorphous substance shows broad peaks (halos) in its powder X-ray diffraction pattern (Ichiro Shinkai, ed., *lyakuhin no Takei Gensho to Shoseki no Kagaku* (or Polymorphism of Pharmaceuticals and Science of Crystallization), Gijutsu-Joho Kyokai, Japan, p.75 (2001))".

Yu L, *Advanced Drug Delivery Reviews,* (England), 48, 27-42 (2001) discloses various processes for producing amorphous substances. Further, the published specification of WO99/34832 discloses a method for producing an amorphous substance, in which a difference between solubilities under different pH conditions is used to rapidly precipitate an amorphous substance. In connection with Compound A, we thoroughly reviewed the methods described in the above-cited references. In most cases, however, what we obtained were merely crystals of Compound A having low solubility. For example, although we examined the method of rapid precipitation by non-solvent addition, the precipitates obtained were crystals having low solubility.

There has been known a method for solubilizing a sparingly soluble substance, in which the substance is made into a coprecipitate. Various references disclose a variety of coprecipitates. For example, the following description is found in *Iwanami Rikagaku Jiten,* 4^{th} ed., Iwanami-Shoten, Japan, p. 310 (1993): coprecipitation is "a phenomenon that, when a certain substance is precipitated from a solution in which solutes having somewhat similar chemical properties coexist, another substance that never precipitates as long as it is present alone precipitates simultaneously with a main precipitate". The following description is also found in *Hirokawa Yakkagaku Dai-jiten,* Hirokawa-Shoten, Japan, p. 331 (1983): coprecipitation is that "when a precipitator is added to a certain ion to produce a precipitate, this ion precipitates together with another coexisting ion that never precipitates when it exists singly". This dictionary also describes as follows: "a coprecipitate is two or more substances that have been simultaneously precipitated from a solution containing the two or more substances and settled due to a change in pH or in conditions for antisolvent addition or for solvent removal by distillation. With respect to combinations of polymers such as povidone and medicines, non-crystallization and improvement in the rate of dissolution are now under study.

More specifically, there are reports on various coprecipitates such as sulfathiazole-polyvinylpyrrolidone coprecipitate (Simonelli A.P., *et al., Journal of Pharmaceutical Sciences,* (USA), 58, 538-549 (1969)). Examples of methods for producing coprecipitates include a method in which a thermoplastic polymer compound and a medicine are thermally melted (melting method), and a method in which a polymer compound and a medicine are dissolved in an organic solvent, and the organic solvent is then removed by vacuum distillation (solvent-distilling-off method). The amount of the polymer compound used in these methods is usually 3 to 10 times the amount of the medicine by weight basis. We tried to apply these methods to a crystalline material of Compound A. For example, we mixed Compound A with Macrogol 6000, a thermoplastic polymer compound, and subjected the mixture to melting with heating, but failed to dissolve Compound A. Further, we dissolved a polymer compound and a crystalline material of Compound A in an organic solvent, and then removed the solvent from the solution by vacuum distillation. However, in the course of concentration, Compound A firstly precipitated as crystals having low solubility.

### SUMMARY OF THE INVENTION

We have now succeeded in obtaining a coprecipitate of Compound A and a water-soluble polymer, excellent in solubility and absorbability. The present invention is based on this finding.

An object of the present invention is, therefore, to provide a coprecipitate of Compound A and a water-soluble polymer, excellent in both solubility and absorbability.
According to the present invention, there can be obtained significantly high water solubility of Compound A. Further, according to the present invention, there can be obtained significantly high bioavailability of Compound A.

### BRIEF DESCRIPTION OF THE DRAWINGS

Fig. 1 is a diagram showing the solubility of the coprecipitate obtained in Example 7, and those of the crystalline materials of Compound A obtained in Comparative Examples 1 and 2 (test medium: water),
Fig. 2 is a chart of the thermal analysis (DSC) of the coprecipitate obtained in Example 7,
Fig. 3 is a chart of the thermal analysis (DSC) of the crystalline material of Compound A obtained in Comparative Example 1,
Fig. 4 is a chart of the thermal analysis (DSC) of the crystalline material of Compound A obtained in Comparative Example 2,
Fig. 5 shows a powder X-ray diffraction pattern of the coprecipitate obtained in Example 7, and those of the crystalline materials of Compound A obtained in Comparative Examples 1 and 2, and
Fig. 6 is a diagram showing changes in the Compound B content of blood plasma, obtained by orally administering, to cynomolgus monkeys, a suspension prepared by suspending, in 1% methyl cellulose, the coprecipitate obtained in Example 7 or the crystalline material of Compound A obtained in Comparative Example 1.

### DETAILED DESCRIPTION OF THE INVENTION

A coprecipitate according to the present invention has broad peaks at the diffraction angles (2θ) in the vicinity of: 4.6°, 10.5°, and 26.0° in a powder X-ray diffraction pattern. These diffraction peaks are characteristic of the coprecipitate according to the present invention. Further, the coprecipitate according to the present invention has a broad exothermic peak in the range of 120 - 180°C and a sharp endothermic peak in the range of 220 - 230°C in a thermal analysis using a differential scanning calorimetry. As far as we know, a coprecipitate including Compound A, having such physicochemical properties, has not yet been known. It can, therefore, be safely said that this coprecipitate is novel. The solubility, in water at 37°C, of the coprecipitate according to the present invention is 14 - 20 µg/mL, as indicated by the concentration of Compound A in water.

Examples of water-soluble polymers useful for the present invention include cellulosic water-soluble polymers, and, more specifically, include methyl cellulose, hydroxypropylmethyl cellulose, hydroxypropyl cellulose, and hydroxyethyl cellulose and the like. Of these, methyl cellulose or hydroxypropylmethyl cellulose is preferred. Therefore, according to the preferred embodiment of the present invention, the water-soluble polymer is a cellulosic water-soluble polymer.

In the coprecipitate according to the present invention, the weight mixing ratio of Compound A and the water-soluble polymer is preferably from 1:0.05 to 1:1, more preferably from 1:0.1 to 1:0.5. As long as the coprecipitate of the invention is produced in the above-described the weight mixing ratio, the viscosity of the water-soluble polymer does not impede the production of the coprecipitate, and, moreover, when the coprecipitate according to the present invention is formulated into a solid preparation for oral administration, the preparation can have proper disintegrating and dissolving properties.

Furthermore, the coprecipitate according to the present invention can be used for the prophylaxis or treatment of allergic diseases. Allergic diseases herein include bronchial asthma, eczema, urticaria, allergic gastrointestinal disorders, allergic rhinitis, and allergic conjunctivitis. Therefore, according to another aspect of the present invention, the coprecipitate of the invention is used as a pharmaceutical bulk. The present invention also provides, in a further aspect, an allergic medicine comprising the coprecipitate according to the present invention. In addition, the present invention provides, in a still further aspect, a composition, especially a pharmaceutical composition, comprising the coprecipitate according to the present invention.

For oral administration, the coprecipitate of the present invention, or the composition of the present invention may be formulated, together with known pharmaceutically acceptable excipients (e.g., lactose, crystalline cellulose, starch, calcium phosphate, etc.), binders (e.g., starch, sodium carmellose, hydroxypropyl cellulose, etc.), disintegrants (calcium carmellose, Crosscarmellose sodium, Crosspovidone, etc.), lubricants (magnesium stearate, talc, etc.), and so on, into tablets, capsules, granules, and dry syrups that are commonly used for medical purposes, or may be formulated into a variety of liquid agents including syrups by conventional methods. Further, a variety of these preparations may also be presented as sustained-release preparations that act for a prolonged period of time. Therefore, according to another aspect of the present invention, there is provided a pharmaceutical composition for oral administration, comprising the coprecipitate of the present invention and a pharmaceutically acceptable carrier.

As can be clearly known from the above, the present invention provides, in another aspect, use of the coprecipitate according to the present invention for the production of a pharmaceutical composition. The present invention also provides, in a further aspect, use of the coprecipitate according to the present invention for the production of an antiallergic medicine. Moreover, the present invention provides, in a still further aspect, a method for preventing or treating an allergic disease, comprising administering the coprecipitate according to the present invention to an animal including a human.

In the production of the coprecipitate according to the present invention, Compound A in the form of a crystalline material is usually used. A crystalline material of Compound A according to the present invention can be obtained in the following manner, for example: Compound A is dissolved in methylene chloride at a temperature between 15°C and 30°C; methanol is added to this solution; and Compound A is recrystallized from this mixture.

Another process is as follows. Compound A is dissolved in at least one organic solvent selected from N,N-dimethylformamide, dimethyl sulfoxide, N,N-dimethylacetamide, and N-methyl-2-pyrrolidone, at a temperature between 20°C and 90°C. The solution is filtered, if desired, and is then added dropwise to stirred water at 0 to 30°C, and the precipitate is collected by filtration. This precipitate is washed with water, if desired, and is then dried under reduced pressure to give a crystalline material of Compound A according to the present invention.

According to the preferred embodiment of the present invention, there can be obtained the coprecipitate of the invention by the following process. In the production process according to the present invention, a water-soluble organic solvent solution containing Compound A and a liquid medium containing water as a main component are firstly prepared. A water-soluble polymer to be coprecipitated with Compound A is then dissolved at least in the water-soluble organic solvent solution containing Compound A or in the liquid medium containing water as a main component. Preferably, both the water-soluble organic solvent solution containing Compound A and the liquid medium containing water as a main component contain the water-soluble polymer. The water-soluble organic solvent solution containing Compound A is mixed with the liquid medium containing water as a main component to give a mixture,wherein a coprecipitate is produced. The coprecipitate produced is isolated by a conventional method such as filtration or centrifugal separation. Therefore, the present invention provides, in another aspect, a process for producing the coprecipitate of the present invention. This process comprises the steps of mixing a water-soluble organic solvent solution containing Compound A with a liquid medium containing water as a main component to give a mixture, wherein a coprecipitate is produced, and isolating the coprecipitate from the mixture. The water-soluble organic solvent solution containing Compound A and/or the liquid medium containing water as a main component comprise/comprise a water-soluble polymer. This process is hereinafter referred to as the "batch process".

Examples of aqueous organic solvents useful for this batch process include dimethyl sulfoxide, N,N-dimethylformamide, N,N-dimethylacetamide, and N-methyl-2-pyrrolidone and the like. Therefore, according to the preferred embodiment of the present invention, the aqueous organic solvent is dimethyl sulfoxide, N,N-dimethylformamide, N,N-dimethylacetamide or N-methyl-2-pyrrolidone.

The concentration of Compound A in the water-soluble organic solvent solution is preferably 10 to 30 w/v%, more preferably 15 to 20 w/v%.

When the water-soluble organic solvent solution contains a water-soluble polymer, the concentration of the water-soluble polymer in the water-soluble organic solvent solution is preferably 0 to 45 w/v%, more preferably 10 to 40 w/v%.

Further, when the water-soluble organic solvent solution contains a water-soluble polymer, the weight mixing ratio of Compound A and the water-soluble polymer in the water-soluble organic solvent solution is preferably from 1:0.05 to 1:1, more preferably from 1:0.1 to 1:1.

Although there are no restrictions on the temperature of the water-soluble organic solvent solution at the time when Compound A and the water-soluble polymer are dissolved in the organic solvent, and at the time when the water-soluble organic solvent solution is mixed with the liquid medium containing water as a main component, this temperature is preferably 20°C or more, more preferably 60°C or more.

In the case where the above mixture contains a water-soluble polymer, although the liquid medium containing water as a main component can be simply water, it is preferred that the liquid medium be an aqueous solution containing the water-soluble polymer.

When the liquid medium containing water as a main component contains a water-soluble polymer, the concentration of the water-soluble polymer in the liquid medium is preferably less than 7 w/v%, more preferably 0.05 to 5 w/v%.

Further, in order to increase the efficiency of precipitation of the coprecipitate and also to make the quality of the resulting coprecipitate uniform, it is preferable to cool the liquid medium containing water as a main component before it is mixed with the water-soluble organic solvent solution. Although there are no restrictions on the temperature to which the liquid medium is cooled, it is preferably 15°C or less.

In the production process according to the present invention, there are no restrictions on the mixing ratio of the water-soluble organic solvent solution to the liquid medium containing water as a main component. However, letting the volume of the water-soluble organic solvent solution equal to 1, the volume of the liquid medium containing water as a main component is preferably from 3 to 100, more preferably from 5 to 20.

In the production process according to the present invention, the liquid medium containing water as a main component may be added to the whole amount of the water-soluble organic solvent solution containing Compound A. In contrast with this, the water-soluble organic solvent solution containing Compound A may be added to the whole amount of the liquid medium containing water as a main component. It is preferable to mix the two solutions with stirring. Although there are no restrictions on the rate of stirring, it is preferably from 50 to 300 rpm.

It is also possible to produce the coprecipitate of the invention by the following process. According to this process, a water-soluble organic solvent solution containing Compound A and a liquid medium containing water as a main component are prepared so that their compositions are the same as those described above. The water-soluble organic solvent solution containing Compound A is then introduced into a tube in which the liquid medium containing water as a main component flows. Thus, the water-soluble organic solvent solution and the liquid medium form a mixture that flows in the tube at a constant rate. From this mixture, a coprecipitate according to the present invention is produced. By a conventional method such as filtration or centrifugal separation, the coprecipitate of the invention can be isolated from the mixture that is discharged from the tube. This process is hereinafter referred to as the "in-line process".

The in-line process is specifically as follows. The liquid medium containing water as a main component is introduced into a first tube (inner diameter: 2.5 - 100 mm) at a rate of 60 to 3600 mL/min. The aqueous organic solvent solution containing Compound A is prepared and is then introduced into a second tube (inner diameter: 0.25 to 10 mm) at a rate of 6 to 360 mL/min. From the injection point at which the second tube is connected to the first tube, the aqueous organic solvent solution is injected into a stream of the liquid medium containing water as a main component, thereby obtaining a mixture of the two solutions whose flow rate is from 60 to 3600 mL/min and producing a precipitate from this mixture. The mixture is discharged from the end of the first tube. The length of the first tube, between the injection point and the point from which the mixture is discharged, is preferably from 5 to 50 m. The mixture discharged is subjected to filtration, centrifugal separation, or the like. Thus, there can be obtained the coprecipitate according to the present invention.

The in-line process is advantageous in that the water-soluble organic solvent solution containing Compound A and the liquid medium containing water as a main component can always be kept in contact with each other under certain conditions and that a homogeneous coprecipitate can, therefore, be continuously produced with high efficiency. Further, with the in-line process, it is possible to prevent the coprecipitate from undergoing fine powdering that is caused by mechanical pulverization such as stirring. The in-line process is thus advantageous also in that it is easy to effect the succeeding steps, such as the step of collecting the coprecipitate by filtration.

Since the water-soluble organic solvent remains in the coprecipitate obtained by the batch process or the in-line process, it is preferable to remove some of the solvent before treating the coprecipitate in the drying step. To remove the water-soluble organic solvent, there can be employed a method in which the following steps are repeated, as needed: the coprecipitate collected by filtration is dispersed again in an aqueous solution of a water-soluble polymer compound or in water, and the dispersed coprecipitate is collected by filtration. The following method can also be employed particularly in the in-line process: a suspension containing the coprecipitate is introduced directly into a centrifugal solid-liquid separator, to which a fresh liquid medium containing water as a main component is introduced to remove the water-soluble organic solvent. Preferably, the composition of this liquid medium containing water as a main component is the same as that of the liquid medium containing water as a main component that has been used in the production process of the invention. The dehydrated cake obtained is then dried by a conventional method such as vacuum drying or freeze-drying. Thus, there can be finally obtained the coprecipitate according to the present invention.

### EXAMPLES

The present invention will now be explained more specifically by referring to the following Examples. However, the present invention is not limited to these Examples.
The composition of the coprecipitate according to the present invention, comprising Compound A and the water-soluble polymer compound, is indicated by the Compound A content (mass percentage %). The solubility herein means the concentration of Compound A in a suspension obtained by suspending, in water (37°C), the coprecipitate obtained in each Example or the crystalline material of Compound A obtained in each Comparative Example. The concentration of Compound A (µg/mL) is herein represented by the concentration of Compound A (µg/mL) at the point of 30 minutes in the method described in Test 1.

Comparative Example 1
The light yellow powder obtained in accordance with the process described in Example 20 in the published specification of WO99/16770 was dissolved in methylene chloride and was recrystallized from methanol to give a crystalline material of Compound A. This material showed the characteristic diffraction peaks in powder X-ray diffractometry, and its solubility was 0.8 µg/mL.

Comparative Example 2
The crystalline material of Compound A (0.9 g) obtained in Comparative Example 1 was dissolved in 5.1 mL of dimethyl sulfoxide (hereinafter referred to as "DMSO"). This solution was added dropwise to stirred water (180 mL), and the precipitate was collected by filtration. This precipitate was dispersed in water (90 mL) and was then collected again by filtration. The material collected was dried in a tray-type vacuum freeze-dryer to give a crystalline material of Compound A (0.66 g, solubility: 2.4 µg/mL).

Example 1
The crystalline material of Compound A (9.0 g) obtained in Comparative Example 1 and methyl cellulose (Metolose SM15, manufactured by SHIN-ETSU CHEMICAL CO., LTD., Japan, 1.8 g) were dissolved in 51 mL of DMSO. This solution was added dropwise to a 0.5% aqueous methyl cellulose solution (300 mL) with stirring, and the precipitate was collected by filtration. This precipitate was dispersed in a 0.5% aqueous methyl cellulose solution (100 mL) and was collected again by filtration. The material collected was dried in a tray-type vacuum freeze-dryer (Model C-12-3-ST, manufactured by Birches Corp.) to give a coprecipitate of methyl cellulose and Compound A (6.0 g, content: 81.9%, solubility: 16.8 µg/mL).

Example 2:
The crystalline material of Compound A (900 mg) obtained in Comparative Example 1 and 180 mg of hydroxypropylmethyl cellulose (TC-5R, manufactured by SHIN-ETSU CHEMICAL CO., LTD., Japan, hereinafter referred to as "HPMC") were dissolved in 8.6 mL of N,N-dimethylformamide (hereinafter referred to as "DMF"). This solution was added dropwise to a 0.5% aqueous HPMC solution (270 mL) with stirring, and the precipitate was collected by filtration. This precipitate was dispersed again in a 0.5% aqueous HPMC solution (100 mL) and was then collected by filtration. The material collected was dried in the tray-type vacuum freeze-dryer to give a coprecipitate of HPMC and Compound A (720 mg, content: 80.2%, solubility: 15.8 µg/mL).

Example 3:
The crystalline material of Compound A (900 mg) obtained in Comparative Example 1 was dissolved in DMF (8.6 mL). This solution was added dropwise to a 0.5% aqueous HPMC solution (270 mL) with stirring, and the coprecipitate produced was collected by filtration. This coprecipitate was dispersed again in a 0.5% aqueous HPMC solution (270 mL), and the dispersion was filtered. The residue was dried in the tray-type vacuum freeze-dryer to give a coprecipitate of HPMC and Compound A (600 mg, content: 82.9%, solubility: 16.6 µg/mL).

Example 4:
The crystalline material of Compound A (30.0 g) obtained in Comparative Example 1 and HPMC (6.0 g) were dissolved in DMSO (170 mL). This solution was added dropwise to a 0.5% aqueous HPMC solution (1000 mL) with stirring, and the precipitate was collected by filtration. This precipitate was dispersed again in a 0.5% aqueous HPMC solution (300 mL) and was then collected by filtration. The material collected was dried in the tray-type vacuum freeze-dryer to give a coprecipitate of HPMC and Compound A (20 g, content: 81.6%, solubility: 15.8 µg/mL).

Example 5
The crystalline material of Compound A (0.9 g) obtained in Comparative Example 1 and HPMC (0.18 g) were dissolved in DMSO (5.1 mL). This solution was added dropwise to water (180 mL) with stirring, and the precipitate was collected by filtration. This precipitate was dispersed again in water (90 mL) and was then collected by filtration. The material collected was dried in the tray-type vacuum freeze-dryer to give a coprecipitate of HPMC and Compound A (0.72 g, content: 89.1%, solubility: 16.2 µg/mL).

Example 6
The crystalline material of Compound A (0.9 g) obtained in Comparative Example 1 was dissolved in DMSO (5.1 mL). This solution was added dropwise to a 0.5% aqueous HPMC solution (180 mL) with stirring, and the precipitate was collected by filtration. This precipitate was dispersed again in a 0.5% aqueous HPMC solution (90 mL) and was then collected by filtration. The material collected was dried in the tray-type vacuum freeze-dryer to give a coprecipitate of HPMC and Compound A (0.63 g, content: 86.2%, solubility: 16.3 µg/mL).

Example 7
A 0.5% aqueous HPMC solution was introduced into a first tube (inner diameter: 8 mm, silicone-rubber-made, 30 m) at a rate of 1,800 mL/min. The crystalline material of Compound A obtained in Comparative Example 1 (150 g) and HPMC (30 g) were dissolved in DMSO (840 mL). This DMSO solution was introduced into a second tube (inner diameter: 2.5 mm, stainless-steel-made) at a rate of 60 mL/min. From the injection point at which the second tube was connected to the first tube, the DMSO solution was injected into a stream of the 0.5% aqueous HPMC solution, thereby obtaining a mixture of the two solutions whose flow rate was 1,800 mL/min. The mixture discharged from the end of the first tube was then filtered through a strainer (a sieve No. 100 according to the Japanese Pharmacopoeia). The residue was dispersed in a 0.5% aqueous HPMC solution (4,000 mL), and the dispersion was filtered again through the strainer. The residue was dried in the tray-type vacuum freeze-dryer to give a coprecipitate of HPMC and Compound A (112 g, content: 84.9%, solubility: 15.7 µg/mL).

Example 8
A 1% aqueous HPMC solution was introduced into a first tube (inner diameter: 8 mm, silicone-rubber-made, 30 m) at a rate of 1,800 mL/min. The crystalline material of Compound A obtained in Comparative Example 1 (200 g) and HPMC (40 g) were dissolved in DMSO (1,120 mL). This DMSO solution was introduced into a second tube (inner diameter: 2.5 mm, stainless-steel-made) at a rate of 180 mL/min. From the injection point at which the second tube was connected to the first tube, the DMSO solution was injected into a stream of the 1% aqueous HPMC solution, thereby obtaining a mixture of the two solutions whose flow rate was 1,800 mL/min. The mixture discharged from the end of the second tube was introduced into a centrifugal solid-liquid separator (Model H-110, manufactured by Kokusan Corp., Japan), and the solid material contained in the mixture was separated (1500 rpm, 15 minutes). To this centrifugal solid-liquid separator, a 1% aqueous HPMC solution (15 liters), divided into several portions, was fed one portion after another, and the solid material was centrifugally separated again. This solid material was then dried in the tray-type vacuum freeze-dryer to give a coprecipitate of HPMC and Compound A (213.74 g, content: 83.6%, solubility: 14.9 µg/mL).

Test 1: Solubility test
By using, as test samples, the coprecipitate obtained in Example 7 and the crystalline materials of Compound A obtained in Comparative Examples 1 and 2, a solubility test was carried out, in which water was used as a test medium. Each test sample was prepared in an amount approximately equivalent to 100 mg of Compound A and was added to 500 mL of water (37°C), and the mixture was stirred at 200 rpm with paddles. Each mixture was sampled at several points of time, and each sample was filtered through a membrane filter (Sunprep LCR13-LG, manufactured by Millipore Corp., Japan). The compound A concentration of each filtrate was determined with high-performance liquid chromatography (HPLC).
The measurement conditions used for HPLC in Test 1 were as follows:
- Detector:: Ultraviolet absorptiometer (measurement wavelength: 246 nm)
- Column:: Stainless steel tube with an inner diameter of 4.6 mm and a length of 25 cm, for 5 µm liquid chromatography,
- Column temperature:: Fixed temperature at around 40°C
- Mobile phase:: Mixture of methanol and water (55:45)
- Flow rate:: 1 mL/min

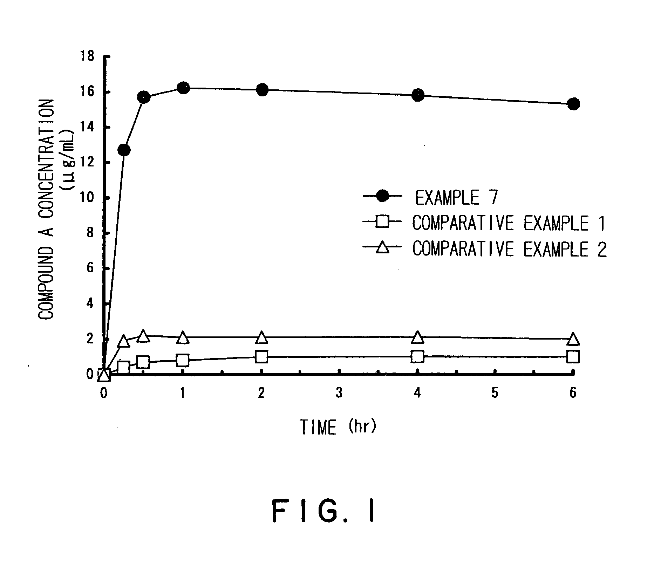

The results were as shown in Fig. 1. The concentration of the coprecipitate obtained in Example 7 was 14 µg/mL or more at 30 minutes after the beginning of the test, and after this point of time. On the other hand, the concentration of the crystalline material of Compound A obtained in Comparative Example 1 and that of the crystalline material of Compound A obtained in Comparative Example 2 were below 3 µg/mL.
Separately from the above, it was confirmed that, even when an aqueous methyl cellulose or HPMC solution (e.g., a 0.5% aqueous solution) was used as the test medium, the solubility of the crystalline material of Compound A obtained in Comparative Example 1 and that of the crystalline material of Compound A obtained in Comparative Example 2 were nearly equal to the above-described value of concentration.

Test 2: Thermal analysis (DSC)
The coprecipitate obtained in Example 7 and the crystalline materials of Compound A obtained in Comparative Examples 1 and 2 were analyzed by the use of a thermal analyzer (DSC). The measurement conditions for this analysis were as follows:
- Equipment:: DSC 7 (manufactured by Perkin Elmer Corp.)
Measurement conditions:
- sample:: 3 - 5 mg, pan: open aluminium pan,
- atmosphere:: nitrogen, gas flow rate: 50 mL/min,
- heating rate:: 5°C/min,
- temperature range:: 75 - 250°C.

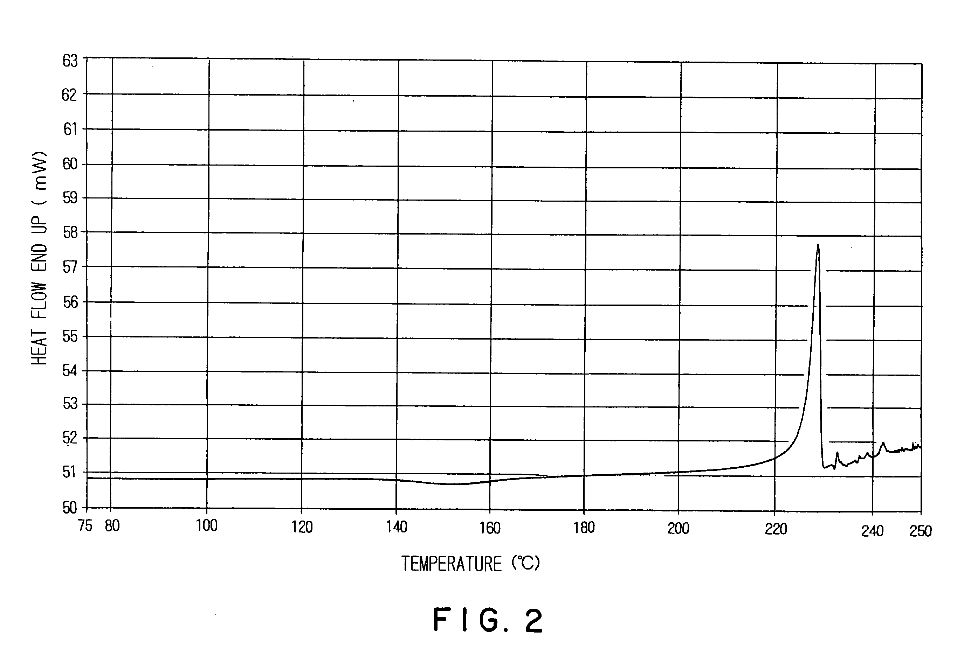

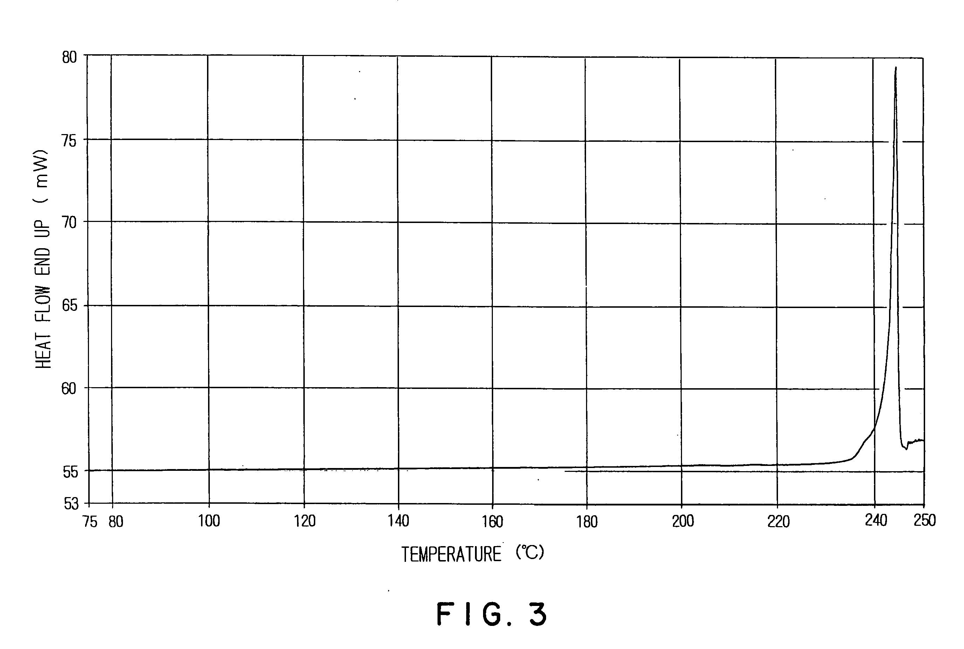

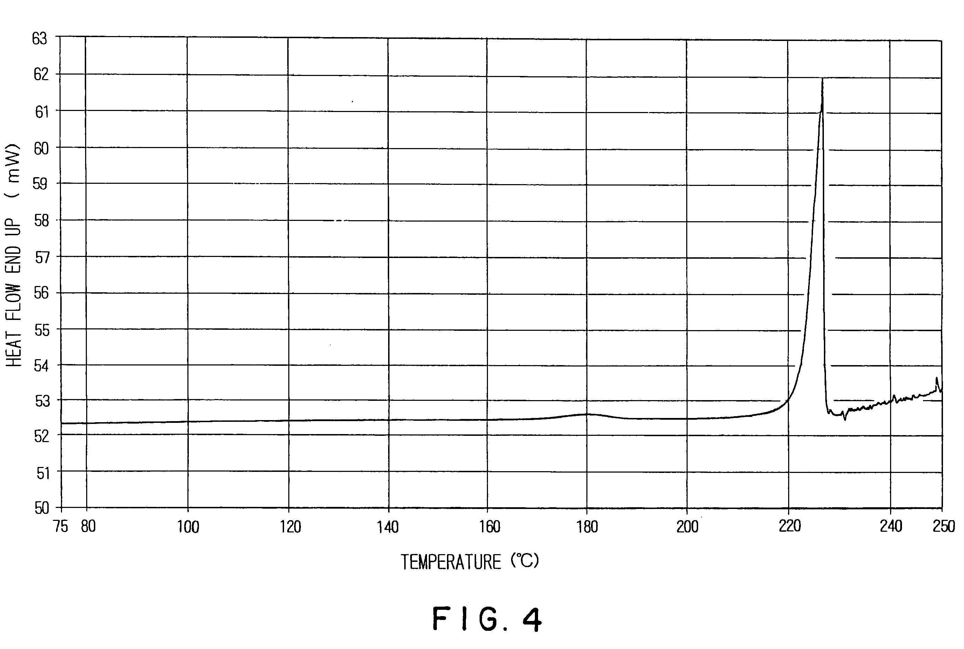

The results were as shown in Figs. 2, 3, and 4. The coprecipitate obtained in Example 7 (Fig. 2) had a broad exothermic peak in the range of 120 - 180°C and a sharp endothermic peak in the range of 220 - 230°C. The crystalline material of Compound A obtained in Comparative Example 1 (Fig. 3) had an endothermic peak in the vicinity of 240°C. The crystalline material of Compound A obtained in Comparative Example 2 (Fig. 4) had two endothermic peaks in the vicinity of 190°C and of 225°C. The crystalline material of Compound A obtained in Comparative Example 1 (Fig. 3) and that obtained in Comparative Example 2 (Fig. 4) had, in their DSC charts, no exothermic peak. Thus, the coprecipitate obtained in Example 7 was different from the crystalline materials of Compound A obtained in Comparative Examples 1 and 2 in that the former had an exothermic peak. The endothermic peak the coprecipitate of Example 7 had was very similar to that the crystalline material of Compound A obtained in Comparative Example 2 had, in temperature at which the peak appeared (around 225°C) and also in calorie per unit mass obtainable from the peak.

Test 3: Powder X-ray diffraction
The coprecipitate obtained in Example 7, and the crystalline materials of Compound A obtained in Comparative Examples 1 and 2 were identified by the use of a powder X-ray diffractometer. The measurement conditions were as follows:
- Equipment:: RINT 2200 (manufactured by Rigaku Corp., Japan)
Measurement conditions:
- X-ray:: CuKα, voltage: 40 kV, current: 20 mA,
- monochrome:: graphite monochromator,
- scanning speed:: 4°/min, scanning step: 0.02°,
- axis of scanning:: 2θ/θ, divergent slit: 1°, scattering slit: 1°,
- photo-receptive slit:: 0.30 mm, scanning range: 2θ = 3 - 40°

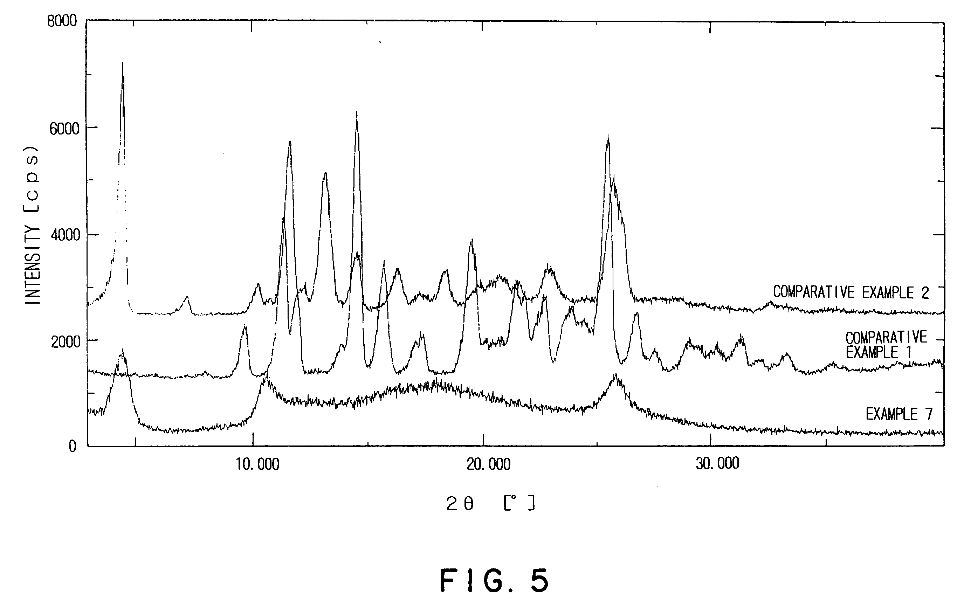

The results were as shown in Fig. 5. The coprecipitate obtained in Example 7 had broad diffraction peaks at the characteristic diffraction angles (2θ) in the vicinity of: 4.6°, 10.5°, and 26.0°, but had no halo patterns. On the other hand, the crystalline materials of Compound A obtained in Comparative Examples 1 and 2 had intense, sharp diffraction peaks at the respective characteristic diffraction angles.

Test 4: Absorbability test
When absorbed into organisms, Compound A is converted into 7,8-dimethoxy-4(5H),10-dioxo-2H-1,2,3-triazolo[4,5-c][1]benzazepine (hereinafter referred to as Compound B), a main body that exhibits the physiological activity of Compound A. The following test was carried out, using Compound B as an indication.

The coprecipitate obtained in Example 7 or the crystalline material of Compound A obtained in Comparative Example 1 was suspended in a 1% aqueous HPMC solution. Each suspension was orally administered to a group of cynomolgus monkeys (n = 4) that had not been fed one overnight (Example 7: 25 mg/kg, Comparative Example 1: 5 mg/kg). The two samples were compared, in terms of the change in the Compound B concentration of blood plasma and the area under the medicine concentration in blood plasma-time curve (AUC), to evaluate the difference in absorbability between the samples. The Compound B concentration of blood plasma derived from the blood sample was determined in the following manner.
<Pretreatment of Blood> Blood plasma was, in the presence of heparin, centrifugally separated (4°C, approximately 1600 x g, 10 minutes) from blood (1 mL) that had been drawn from a saphenous vein. To this blood plasma (100 µL) was added methanol containing an internal standard substance (sodium 7-methyl-4(5H),10-dioxo-2H-1,2,3-triazolo[4,5-c][1]benzazepine) (100 ng/mL, 100 µL), and the mixture was stirred and was then subjected to centrifugal separation (4°C, 8200 x g, 5 minutes). The supernatant liquid was centrifugally evaporated to dryness under reduced pressure. An HPLC mobile phase (150 µL) was added to the residue for redissolution, and the solution obtained was used as a sample for HPLC.

The measurement conditions used for HPLC in Test Example 4 were as follows:
- HPLC pump:: 600E (Nippon Waters Corp., Japan)
- Autosampler:: 717 plus (Nippon Waters Corp., Japan)
- Detector:: RF-10AXL (Shimadzu Corp., Japan)
- Fluorescence detection wavelengths:: Ex 270 nm, Em 466 nm
- Column:: Cosmosil 5C18-AR-II (4.6 x 150 mm, manufactured by Nacalai Tesque, Inc., Japan)
- Guard column:: Cosmosil 5C18-AR (4.6 x 10 mm, manufactured by Nacalai Tesque, Inc., Japan)
- Column temperature:: 35°C
- Mobile phase:: 10 mmol/liter phosphoric acid buffer (pH 7.0) : methanol (4:1)
- Flow rate:: 0.8 mL/min
- Amount of influent:: 20 µL

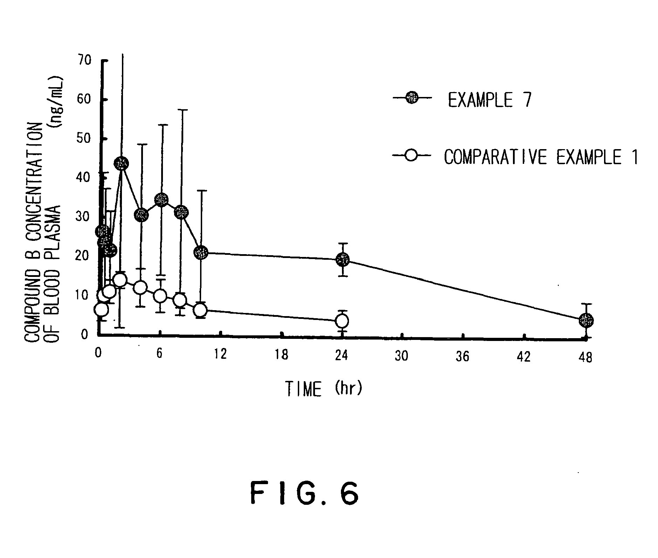

The results were as shown in Fig. 6. In Fig.6, for convenience of comparison, the Compound B concentration of blood plasma, obtained by administering the coprecipitate of Example 7, are normalized to correspond to the amount in which the crystalline material of Comparative Example 1 was administered. The Compound B concentration of blood plasma obtained by administering the coprecipitate of Example 7 were significantly higher than those obtained by administering the crystalline material of Comparative Example 1. Moreover, with respect to the AUCs obtainable from the changes in the Compound B concentration of blood plasma, the AUC obtained by administering the crystalline material of Comparative Example 1 was 170 ± 59 ng.hr/mL, while that obtained by administering the coprecipitate of Example 7 was 894 ± 341 ng·hr/mL. Like the Compound B contents of blood plasma, the AUC of Example 7 is herein normalized to correspond to the amount in which the crystalline material of Comparative Example 1 was administered. The AUC of Example 7 was approximately 5 times greater than that of Comparative Example 1.

## Claims

1. A coprecipitate of 2-(1-isopropoxycarbonyloxy-2-methylpropyl)-7,8-dimethoxy-4(5H),10-dio xo-2H-1,2,3-triazolo[4,5-c][1]benzazepine and a water-soluble polymer.

2. The coprecipitate according to claim 1, which has broad peaks at the diffraction angles (2θ) in the vicinity of: 4.6°, 10.5°, and 26.0° in a powder X-ray diffraction pattern.

3. The coprecipitate according to claim 1 or 2, which has a broad exothermic peak at 120 - 180°C and a sharp endothermic peak at 220 - 230°C in a thermal analysis using a differential scanning calorimetry.

4. The coprecipitate according to any one of claims 1 to 3, which has a solubility, in water at 37°C, of 14 to 20 µg/mL, as indicated by the concentration of 2-(1-isopropoxycarbonyloxy-2-methylpropyl)-7,8-dimethoxy-4(5H),10-dio xo-2H-1,2,3-triazolo[4,5-c][1]benzazepine.

5. The coprecipitate according to any one of claims 1 to 4, wherein the weight mixing ratio of 2-(1-isopropoxycarbonyloxy-2-methylpropyl)-7,8-dimethoxy-4(5H),10-dio xo-2H-1,2,3-triazolo[4,5-c][1]benzazepine and the water-soluble polymer is from 1:0.05 to 1:1.

6. The coprecipitate according to any one of claims 1 to 5, wherein the water-soluble polymer is a cellulosic water-soluble polymer.

7. The coprecipitate according to claim 6, wherein the water-soluble polymer is methyl cellulose or hydroxypropylmethyl cellulose.

8. A pharmaceutical composition for oral administration, comprising the coprecipitate according to any one of claims 1 to 7, and a pharmaceutically acceptable carrier.

9. The coprecipitate according to any one of claims 1 to 7, which is used as a pharmaceutical bulk.

10. An antiallergic medicine comprising the coprecipitate according to any one of claims 1 to 7.

11. A process for producing the coprecipitate according to any one of claims 1 to 7, comprising the steps of:
mixing a water-soluble organic solvent solution containing 2-(1-isopropoxycarbonytoxy-2-methylpropyl)-7,8-dimethoxy-4(5H),10-dio xo-2H-1,2,3-triazolo[4,5-c][1]benzazepine and a liquid medium containing water as a main component to give a mixture, wherein the coprecipitate is produced, and
isolating the coprecipitate from the mixture,
wherein the water-soluble organic solvent solution and/or the liquid medium comprise/comprises a water-soluble polymer.

12. The process for producing the coprecipitate according to claim 11, wherein the water-soluble organic solvent is dimethyl sulfoxide, N, N-dimethylformamide, N, N-dimethylacetamide, or N-methyl-2-pyrrolidone.

13. Use of the coprecipitate according to any one of claims 1 to 7 for the production of a pharmaceutical composition.

14. Use of the coprecipitate according to any one of claims 1 to 7 for the production of an antiallergic medicine.

15. A method for preventing or treating an allergic disease, comprising administering the coprecipitate according to any one of claims 1 to 7 to an animal including a human.
